# EUROPEAN PATENT SPECIFICATION

(11) **EP 1 380 297 B1**
(45) Date of publication and mention of the grant of the patent: **27.05.2009**
(21) Application number: 03023240.9
(22) Date of filing: 08.07.1999
(51) Int. Cl.: A61K 31/445, A61K 9/20

(54) **8-chloro-6,11-dihydro-11-(4-piperidylidene)-5H-benzo[5,6]-cyclo-hepta[1,2-b]pyridine oral compositions**
Orale Zusammensetzungen mit 8-chloro-6,11-dihydro-11-(4-piperidylene)-5H-benzo[5,6]-cyclo-hepta[1,2-b]pyridin
Compositions orales à base de 8-chloro-6,11-dihydro-11-(4-piperidylene)-5H-benzo[5,6]-cyclo-hepta[1,2-b]pyridine

(30) Priority: 10.07.1998 US 113232
(43) Date of publication of application: 14.01.2004
(62) Divisional of application: 99933484.0
(73) Proprietor: Schering Corporation, Kenilworth, NJ 07033-0530 (US)
(72) Inventor: Kou, Jim H., Basking Ridge, NJ 07920 (US)
(74) Representative: Klusmann, Peter

(56) References cited:
- EP-A- 0 396 404
- WO-A-98/34614

## Description

### Background of the Invention

This invention relates to pharmaceutical compositions containing 8-chloro-6,11-dihydro-11-(4-piperidylidene)-5H-benzo[5,6]-cyclohepta[1,2-b]pyridine (hereinafter "descarbonylethoxyloratadine" or ("DCL") and substantially free of DCL decomposition products, and suitable for oral administration to treat allergic reactions in mammals.

U.S. Patent No. 4,659,716 discloses descarbonylethoxy-loratadine which possesses antihistaminic properties with substantially no sedative properties. This U.S. Patent also discloses methods of making descarbonyl-ethoxyloratadine; pharmaceutical compositions it and methods of using the compositions to treat allergic reactions in mammals.

U.S. Patent No 5,595,997 discloses pharmaceutical compositions and methods for treating allergic rhinitis using descarbonylethoxyloratadine. Co-pending, commonly-owned U.S. Patent application Serial No. 08/886,766, filed 07/02/97 discloses polymorphs of descarbonyl-ethoxyloratadine and pharmaceutical compositions containing them.

WO 98/34614 discloses pharmaceutical compositions of descarboethoxyloratadine.

We are aware of no prior art that discloses the pharmaceutical compositions of the present invention.

There is a need to produce pharmaceutical compositions suitable for oral administration to mammals and containing descarbonylethoxy-loratadine having constant chemical and physical properties in accordance with exacting health registration requirements of the U.S. and international health registration authorities, e.g. , the FDA's Good Manufacturing Practices ("GMP") requirements and the International Conference on Harmonization ("ICH") Guidelines.

### Summary of the Invention

We have found that descarbonylethoxyloratadine discolors and decomposes in the presence of excipients disclosed in the prior art. We have discovered that these problems are substantially solved when the use of an acidic excipient is avoided and descarbonylethoxyloratadine is combined with a pharmaceutically acceptable carrier medium comprising a DCL-protective amount of a pharmaceutically acceptable basic salt. Thus, this invention provides a pharmaceutical composition comprising an anti-allergic effective amount of descarbonylethoxyloratadine in a pharmaceutically acceptable carrier medium comprising a DCL-protective amount of a pharmaceutically acceptable basic salt as defined in claim 1.

The pharmaceutical compositions of the present invention contain less than about 1 % of decomposition products such as N-formylDCL initially, as well as when such compositions are stored at 25°C and 60 % relative humidity for period of at least 24 months.

In a preferred embodiment, this invention provides a pharmaceutical composition comprising an anti-allergic effective amount of descarbonylethoxy-loratadine in a pharmaceutically acceptable carrier medium wherein said composition contains less than 1% by weight of N-formyl DCL, preferably less than 0.8% of N-formyl DCL, and more preferably less than 0.6% of N-formyl DCL.

In another preferred embodiment, this invention provides a pharmaceutical composition for oral administration comprising an anti-allergic effective amount of descarbonylethoxyloratadine in a pharmaceutically acceptable carrier medium comprising a DCL-protective amount of a pharmaceutically acceptable basic salt and an amount of at least one disintegrant sufficient to provide dissolution of at least 80% by weight of the pharmaceutical composition in 45 minutes.

This invention also provides a pharmaceutical composition for oral administration comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least 80% by weight of the pharmaceutical composition in 45 minutes.

In a preferred embodiment, this invention provides a pharmaceutical composition for oral administration comprising an anti-allergic effective amount of descarbonylethoxyloratadine in a pharmaceutically acceptable carrier medium comprising a DCL- protective amount of a calcium dibasic phosphate, and an amount of microcrystalline cellulose and of starch sufficient to provide dissolution of at least 80% by weight of the pharmaceutical composition in 45 minutes, and which contains less than 1% by weight of N-formyldescarbonyl-ethoxyloratadine.

This invention further provides a preferred pharmaceutical composition for oral administration comprising:

| Ingredient | Amount (weight %) |
|---|---|
| Descarbonylethoxyloratadine | 0.5 - 15 |
| Calcium Dibasic Phosphate | |
| Dihydrate USP | 10 - 90 |
| Microcrystalline Cellulose NF | 5 - 60 |
| Corn starch NF | 1 - 60 |
| Talc USP | 0.5 - 20 |

This invention also provides another preferred pharmaceutical composition for oral administration comprising:

| Ingredient | Amount (weight %) |
|---|---|
| Descarbonylethoxyloratadine | 0.5 - 15 |
| Calcium Dibasic Phosphate Dihydrate USP | 45 - 60 |
| Microcrystalline Cellulose NF | 20 - 40 |
| Corn starch NF | 5 - 15 |
| Talc USP | 1-10 |

This invention also provides another preferred pharmaceutical composition for oral administration comprising:

| Ingredient | Amount (weight %) |
|---|---|
| Descarbonylethoxyloratadine | 1 - 10 |
| Calcium Dibasic Phosphate Dihydrate USP | 50 - 56 |
| Microcrystalline Cellulose NF | 25 - 35 |
| Corn Starch NF | 10 - 12 |
| Talc USP | 2 - 5 |

The pharmaceutical compositions of the present invention are useful for treating allergic reactions in mammals.

### Detailed Description of the Invention

During the development of the compositions of the present invention, descarbonylethoxyloratadine was found to discolor when stored at 75% relative humidity ("RH") and a temperature of 40°C, alone or in combination with various excipients, such as those disclosed in U.S. Patent Nos. 4,657,716 and 5,595,997. We discovered that this color instability in the active ingredient was apparently due to a very minute amount of a degradation product caused by the presence of a wide variety of excipients commonly used in oral formulations - especially a tablet formulation. These excipients found unsuitable include acidic excipients including, but not limited to, stearic acid, povidone, and crospovidone, and other acidic excipients having a pH in water fewer than 7, and preferably in the range of about 3 to 5 as well as other excipients such as lactose, lactose monohydrate, sodium benzoate, and Glyceryl Behenate NF sold under the tradename of Compritol 888 The presence of acidic excipients such as stearic acid in a solid powder formulation blend (similar to that of Example 6) containing DCL, lactose monohydrate, and stearic acid resulted in a large amount (14%) of decomposition of descarbonylethoxyloratadine after one week at 40°C and 75 % RH. When the pharmaceutical compositions of the present invention were subjected to the same stressed conditions for a longer period of time, i.e., 3 months, less than about 1% decomposition of descarbonylethoxyloratadine was found in the pharmaceutical compositions of the present invention. See Examples 1-5 and 10 hereinafter. Preferably, the pharmaceutically acceptable carrier medium used in the pharmaceutical compositions of the present invention should be substantially. free, i.e., contain less than 1 % by weight, of acidic excipients.

The major decomposition product of DCL found in the pharmaceutical compositions of the present invention is N-formylDCL. The pharmaceutical compositions of the present invention contain less than 1 % by weight, initially and at periods up to at least 24 months. Preferably, the pharmaceutical compositions of the present invention contain less than 0.8 % by weight, and more preferably they less than 0.6 % by weight of N-formylDCL when such compositions were stored at 25°C and 60 % RH for at least 24 months.

The term"pharmaceutically acceptable basic salts" as used herein means a calcium, magnesium or aluminum salt, or mixtures thereof, including, but not limited to carbonates, phosphates, silicates and sulfates of calcium, magnesium and aluminum. Typically suitable pharmaceutically acceptable basic salts include calcium sulfate anhydrous, hydrates of calcium sulfate, such as calcium sulfate dihydrate, magnesium sulfate anhydrous, hydrates of magnesium sulfate, dibasic calcium phosphate, dibasic calcium phosphate anyhdrous, tribasic calcium phosphate, calcium silicate, magnesium silicate, magnesium trisilicate, aluminum silicate, and magnesium aluminum silicate. The use of calcium phosphate salts is preferred. The use of dibasic calcium phosphate hydrates is more preferred The use of dibasic calcium phosphate dihydrate is most preferred.

The DCL-protective amount of the pharmaceutically acceptable basic salt used in the compositions of the present invention is normally about 50% by weight of the total composition. The w/w ratio of the protective amount of the pharmaceutically acceptable basic salt to the anti-allergic amount of DCL is in the range of 5:1 to 60:1, preferably 7:1 to 11:1, and most preferably 10:1 to 11:1.

The term "disintegrant" as used herein means a pharmaceutically acceptable material or combination of such materials that provides a pharmaceutically acceptable dissolution rate for the compositions of the present invention, preferably a dissolution rate for the compositions of the present invention of at least about 80% by weight in about 45 minutes in accordance with the USP paddle dissolution test <711> on page s 1791-1793 of USP 23/ NF 18, 1995, UNITED STATES PHARMA-COPEIAL CONVENTION, INC., Rockvile MD 20852. Normally, the dissolution rate is measured in 0.1N HCl at 37°C. The preferred dissolution rate of the compositions of the present invention is at least 80 % by weight in 30 minutes, and more preferably, the dissolution rate of the compositions of the present invention is at least 90 % by weight in 30 minutes.

Typically suitable pharmaceutically acceptable disintegrants include microcrystalline cellulose, starch, e.g., pregelatinized starch and corn starch, mannitol, croscarmellose sodium and confectioner's sugar (a mixture of at least 95% by weight sucrose and corn starch that has been ground to a fine powder).The pharmaceutical compositions of the present invention contain at least one, preferably at least two, and most preferably two pharmaceutically acceptable disintegrants in the w/w ratio of 1:1 to 3:1. In a preferred embodiment of the present invention, the two pharmaceutically acceptable disintegrants are cellulose, and starch, preferably corn starch, in the w/w ratio of about 2:1 to about 3:1.

The w/w ratio of the protective amount of the pharmaceutically acceptable basic salt to the amount of the pharmaceutically acceptable disintegrant(s) is in the range of 1.1:1 to 2:1, preferably 1.2:1 to about 1.75:1, and most preferably about 1.20:1 to about 1.25:1.

Unexpectedly, we discovered that when descarbonyl-ethoxyloratadine was combined with a carrier medium comprising dibasic calcium phosphate, and microcrystalline cellulose-in the absence of prior art excipients such as stearic acid, or lactose - we produced a pharmaceutical composition that was stable to discoloration when stored for 4 weeks in open petri dishes at a temperature of 40°C and relative humidity of 75%. In a preferred embodiment of the present invention, the carrier medium also contains corn starch and talc. In place of the corn starch one may substitute pregelatinized starch; the talc may be replaced by PEG 8000. The calcium dibasic phosphate may be replaced by calcium sulfate dihydrate, but use of calcium dibasic phosphate is preferred. No significant changes (less than about 1-2% by weight) were observed in the physical appearance, moisture content, chemical assay of descarbonylethoxyloratadine and dissolution rate of the tablet formulations when a preferred embodiment of the present invention of Example 10 was stored in plastic bottles or blister packages for up to 9 months at 25°C/60% RH or at 30°C/60 % RH or for up to 6 months at 40°C/75 % RH.

The descarbonylethoxyloratadine used in the present invention may be prepared in accordance with Example VI of U.S. Patent No. 4,659,716. Descarbonylethoxyloratadine exists in two polymorphic forms (form 1 and form 2) which may be prepared in accordance with the Examples 1-3 and procedures of commonly-owned co-pending U.S. Patent Application Serial No. 08/886,766 filed 07/02/97. These two polymorphic forms interconverted during the manufacture of the tablets formulation of the present invention. While either polymorph form may be used, form I is preferred.

### Pharmaceutical Compositions

Pharmaceutical compositions of this invention contain an anti-allergically effective amount of descarbonylethoxyloratadine as the active ingredient, and a pharmaceutically acceptable carrier medium which may include, in addition to specific amounts of calcium dibasic phosphate and microcrystalline cellulose, other inert pharmaceutically acceptable ingredients that may be solids or liquids. Solid form compositions include powders, tablets, dispersible granules, capsules, cachets, and suppositories. The inert pharmaceutically acceptable carrier medium includes one or more substances which may also act as diluents, flavoring agents, solubilizers, lubricants, suspending agents, binders, tablet disintegration agents or encapsulating materials. The solid dosage forms of the pharmaceutical compositions of the present invention are suitable for oral administration and include powders, tablets, dispersible granules, capsules, cachets, buccals, and suppositories. In powders, the carrier medium is a finely divided solid which is in admixture with the finely divided active ingredient. In the tablet, the active ingredient is mixed with carrier medium having the necessary binding properties in suitable proportions and compacted in the shape and size desired. The anti-allergic effective amount of DCL in the pharmaceutical compositions of this invention, e.g., powders and tablets is from 0.5 to 15 percent, preferably 0.5 to 10 weight percent, and more preferably 1 to 10 weight percent. The term "compositions" is intended to include the formulation of the active compound with encapsulating material as carrier providing a capsule in which the active ingredient (with or without other carriers) is surrounded by carrier medium, which is thus in association with it. Similarly, caches are included.

The anti-allergic effective amount of descarbonylethoxy-loratadine for oral administration varies from 1 to 50 mg/day, preferably 2.5 to 20 mg/day and more preferably 5 to 10 mg/day in single or divided doses. The most preferred amount is 5 mg, once a day.

Of course the precise dosage and dosage regimen may be varied depending upon the requirements of the patients (e.g.. his or her sex, age) as well as the severity of the allergic condition being treated. Determination of the proper dosage and dosage regimen for a particular patient will be within the skill of the attending clinician.

Descarbonylethoxyloratadine possess antihistaminic properties. These antihistaminic properties have been demonstrated in standard animal models, such as prevention of histamine - induced lethality in guinea pigs. Antihistaminic activity of polymorph form 1 and form 2 of descarbonyl-ethoxyloratadine has also been demonstrated in a monkey model.

### General Experimental

Descarbonylethoxyloratadine may be prepared in accordance with Example VI of USP No. 4,659,716. Calcium dibasic phosphate dihydrate [Ca(H₂PO₄)₂•2H₂O] is available from Rhone Poulenc Rorer, Shelton, CT 06484; microcrystalline cellulose is available from FMC Corporation Food & Pharmaceutical Products, Philadelphia, PA 19103, corn starch NF is available from National Starch & Chemical Corp., Bridgewater, NJ 08807 and the talc USP is available from Whittaker, Clark and Daniels, Inc., South Plainfield, NJ 07080.

### Method of Manufacture of Pharmaceutical Compositions of the Present Invention in the form of Tablets

The following procedure illustrates the formulation of tablets:

### Starch paste preparation

1. Prepare a 10 w/w starch paste by dispersing the paste portion of corn starch into a portion of purified water in a suitable container equipped with an agitator.
2. While mixing, heat the contents of the container to 95°C and maintain this temperature for 30 minutes.
3. Add an additional amount of purified water to the heated mixture and allow the so-formed starch paste to cool to approximately 50°C.
4. While mixing, add the descarbonylethoxyloratadine to the starch paste.

### Granulation

5. To a suitable fluid bed processing bowl, charge the dibasic calcium phosphate dihydrate, a portion of the corn starch and a portion of the microcrystalline cellulose. Place the processing bowl into a fluid bed processor.
6. Fluidize the powder bed and mix for 3 minutes.
7. Begin granulating the powder by pumping the starch paste of step 4 into the fluidized bed at a suitable spray rate (for a 600,000 tablet batch size, the spray rate was 500 mL/min.) and a bed temperature of 22°C.
8. Continue to dry the granulation at 60°C until the granulation has a final loss on drying of 2% or less.
9. Pass the dried granulation through a suitable sieve or mill.
10. Charge the granulation to a suitable blender and add the requisite amount of the remaining portion of microcrystalline cellulose, corn starch, and talc. Blend for 5 minutes to produce a uniform powder blend.

The resulting blend may be filled into suitable two-piece hard gelatin capsules on a suitable encapsulating machine. The blend may also be compressed to an appropriate size and weight on a suitable tablet machine.

### Tableting

1. Compress the final powder blend on a suitable tablet press with a target tablet weight of 100 mg and hardness of 7-9 s.c.u. (Strong-Cobb Units)

The tablets may be film-coated by charging the compressed tablets into suitable coating equipment having a rotating pan and heater. The tablets on the rotating pan are contacted at a temperature of about 30 - 50°C with a coating solutions formed by dissolving clear or colored coating materials in purified water. After the tablets are completely coated, a polishing powder may be added to the coated tablets to provide polished coated tablets. Alternatively, the colored coating material may be added as a dry powder in step 5 or 10, preferably step 5 of the Granulation phase of the process. It is preferred that the colored coating material is preferably substantially free, i.e., < about 1%, or more preferably completely free of offensive excipients such as lactose.

### Examples 1-5

Follow the above-listed manufacturing procedure using the ingredients listed below and compress the powder blend into tablets.

| Ingredients | 1 mg strength (mg/tab) | 2.5 mg strength (mg/tab) | 5mg strength (mg/tab) | 7.5 mg strength (mg/tab) | 10 mg strength (mg/tab) |
|---|---|---|---|---|---|
| Descarbonyl-ethoxyloratadine | 1 | 2.5 | 5 | 7.5 | 10 |
| Dibasic calcium phosphate dihydrate USP | 53 | 53 | 53 | 53 | 53 |
| Microcrystalline cellullose NF | 32 | 30.5 | 28 | 25.5 | 23 |
| Corn starch NF | 11 | 11 | 11 | 11 | 11 |
| Talc USP | 3 | 3 | 3 | 3 | 3 |
| Total | 100 | 100 | 100 | 100 | 100 |

### REFERENCE EXAMPLES 6-9

The tablet formulations of Examples 6-9 were prepared in accordance with procedure of Examples 1-5.

### Reference Example 6

| Ingredients | mg/tablet |
|---|---|
| Descarbonylethoxyloratadine | 10 |
| Lactose monohydrate | 69 |
| Corn starch | 18 |
| Stearic acid | 2 |
| Silicon dioxide | 1 |

### Reference Example 7

| Ingredients | mg/tablet |
|---|---|
| Descarbonylethoxyloratadine | 10 |
| Lactose monohydrate | 59 |
| Microcrystalline cellulose | 8 |
| Pregelatinized starch | 15 |
| Croscarmellose sodium | 5 |
| Silicon dioxide | 1 |
| Stearic acid | 2 |

### Reference Example 8

| Ingredients | mg/tablet |
|---|---|
| Descarbonylethoxyloratadine | 2.5 |
| Dibasic calcium phosphate | 78.5 |
| Dihydrate | |
| Com starch | 18 |
| Magnesium stearate | 1 |

### Reference Example 9

| Ingredients | mg/tablet |
|---|---|
| Descarbonylethoxyloratadine | 2.5 |
| Microcrystalline cellulose | 10 |
| Mannitol | 71.5 |
| Pregelatinized starch | 15 |
| Magnesium stearate | 1 |

The tablet formulations of Reference Examples 6-9 prepared in accordance with procedure of Examples 1-5 discolored rapidly when they were placed in open petri dishes after less than one week under a temperature of 40°C and a relative humidity of 75%.

### Color stability of formulated tablets of Examples 1-5

The color stability of the above mentioned tablets of Examples 1-5 was studied in open petri dishes under a stressed condition of a temperature of 40°C and 75% relative humidity. After storage in the open petri dishes under this condition for 4 weeks, the tablets of Examples 1-5 were found to be free of discoloration and remained white in color. When descarbonyl-ethoxyloratadine was formulated with other excipients such as lactose and stearic acid and formed into tablets, in accordance with the procedures of Examples 1-5, the tablets of Reference Examples 6-9 discolored rapidly after less than one week under the same storage conditions. A solid powder formulation blend(similar to that of the tablets of Examples 6) containing DCL, lactose monohydrate and stearic acid in the w/w/w/ ratio of 1:7:0.2 also decomposed rapidly after less than one week under the same storage conditions of a temperature of 40°C and 75% relative humidity; the chemical assay for descarbonylethoxyloratadine in this solid powder formulation was about 86% of the initial amount and the color of the formulation was pink.

### Example 10

The procedures of Examples 1-5 were followed except that the formulation of Example 3 was compressed into tablets and filmed coated and polished.

| Ingredients | mg/tablet |
|---|---|
| Descarbonylethoxyloratadine | 5.0 |
| Dibasic calcium phosphate | 53.00 |
| Dihydrate USP | |
| Microcrystalline cellulose NF | 28.00 |
| Corn starch NF | 11.00 |
| Talc NF | 3.00 |
| Film coat(blue) | 6.00 |
| Film coat(clear) | 0.6 |
| Polishing Wax¹ | 0.01 |

| | |
|---|---|
| 1. The polishing wax is a 1:1 w/w mixture of Carnuba wax and white wax. | |

### The stability of formulated tablets of Example 10

Chemical assay, physical properties, and photostability of the formulated tablets of Examples 10 were measured on samples placed in high density polyethylene bottles and blister packages.

No significant changes (<1-2%) were observed in the physical appearance, moisture content, chemical assay of descarbonylethoxyloratadine and dissolution rate when the tablets of Example 10 was stored in plastic bottles or blister packages for up to 9 months at 25°C/60% relative humidity("RH") or at 30°C/60 % RH or for up to 6 months at 40°C/75 % RH. A small amount of degradation, e.g., N-formylDCL, was observed in the tablets stored in bottles (about 0.8%) and in blisters( about 1.2%) at 40°C/75 % RH for 6 months; only about 0.2-0.3% of the degradation product was observed in any samples of the tablets stored in blisters or bottles for 9 months at 25°C/60% or at 30°C/60 % RH. It is expected that the International Conference on Harmonization("ICH") Impurity Guideline for a 5 mg tablet stored for 24 months at 25°C/60% RH or for 12 months at 30°C/60 % RH of 1% by weight of the tablet will be met. When the tablets in an open dish were subjected to ICH light conditions for one week at 25°C, the total amount of decomposition products was 0.34% by weight.

### Example 11

The procedures of Examples 10 were followed except that the Blue lake was added as a dry powder to step 5 of the Granulation phase and the formulation was thereafter compressed into tablets

| Ingredients | mg/tablet |
|---|---|
| Descarbonylethoxyloratadine | 5.0 |
| Dibasic calcium phosphate | 53.00 |
| Dihydrate USP | |
| Microcrystalline cellulose NF | 27.72 |
| Corn starch NF | 11.00 |
| Talc NF | 3.00 |
| FD&C Blue #2 Lake | 0.28 |
| Total | 100.00 |

The formulation of Example 11 is expected to have similar chemical assay, and physical properties and photostability to that observed for the formulation of Example 10.

## Claims

1. A pharmaceutical composition for oral administration comprising an anti-allergic effective amount of descarbonylethoxy-loratadine as the active ingredient in a pharmaceutically acceptable carrier medium comprising a DCL-protective amount of a pharmaceutically acceptable basic salt and at least one pharmaceutically acceptable disintegrant, wherein the w/w ratio of the DCL-protective amount of the pharmaceutically acceptable basic salt to said disintegrant is in the range of 1:1 to 2:1, preferably 1.5:1 to 2:1, and preferably 1.25:1 to 1.75:1.

2. The pharmaceutical composition of claim 1 wherein the at least one pharmaceutically acceptable disintegrant is in an amount sufficient to provide dissolution of at least 80% by weight of the pharmaceutical composition in 45 minutes in accordance with the USP paddle dissolution test <711> on page s 1791-1793 of USP 23/ NF 18, 1995, UNITED STATES PHARMA-COPEIAL CONVENTION, INC., Rockvile MD 20852.

3. The pharmaceutical composition of claim 1 wherein the pharmaceutically acceptable basic salt is a calcium, magnesium or aluminum salt, or mixtures thereof.

4. The pharmaceutical composition of claim 1 wherein the pharmaceutically acceptable basic salt is a calcium phosphate salt.

5. The pharmaceutical composition of claim 1 wherein the pharmaceutically acceptable carrier medium is substantially free of acidic excipients.

6. A pharmaceutical composition of claim 1 which contains less than about 1% by weight of N-formyldescarbonylethoxyloratadine after storage at 25°C and 60% relative humidity for at least 24 months.

7. The pharmaceutical composition of claim 1 wherein the w/w ratio of the DCL-protective amount of the pharmaceutically acceptable basic salt to the anti-allergic effective amount descarbonylethoxyloratadine is the range of 5:1 to 60:1, preferably 7:1 to 11:1, and most preferably 10:1 to 11:1.

8. A pharmaceutical composition according to claims 2 or 6 comprising a DCL-protective amount of calcium dibasic phosphate, an amount of microcrystalline cellulose and of starch sufficient to provide dissolution of at least 80% by weight of the pharmaceutical composition in 45 minutes, in accordance with the USP paddle dissolution test <711> on page s 1791-1793 of USP 23/ NF 18, 1995, UNITED STATES PHARMA-COPEIAL CONVENTION, INC., Rockvile MD 20852.

9. A pharmaceutical composition of claim 8 which comprises:
| Ingredient | Amount (weight %) |
|---|---|
| Descarbonylethoxyloratadine | 0.5 - 15 |
| Calcium Dibasic Phosphate | |
| DihydrateUSP | 10 - 90 |
| Microcrystalline Cellulose NF | 5 - 60 |
| Corn starch NF | 1 - 60 |
| Talc USP | 0.5 - 20 |

10. A pharmaceutical composition of claim 8 which comprises:
| Ingredient | Amount (weight %) |
|---|---|
| Descarbonylethoxyloratadine | 0.5 -15 |
| Calcium Dibasic Phosphate | |
| DihydrateUSP | 45 - 60 |
| Microcrystalline Cellulose NF | 20 - 40 |
| Corn starch NF | 5 - 15 |
| Talc USP | 1 - 10. |

11. A pharmaceutical composition of any preceding claim wherein the amount of descarbonylethoxyloratadine is in the range of 1 to 10 weight percent.

12. A pharmaceutical composition of claim 8 which comprises:
| Ingredient | Amount (weight %) |
|---|---|
| Descarbonylethoxyloratadine | 1 - 10 |
| Calcium Dibasic Phosphate Dihydrate USP | 50 - 56 |
| Microcrystalline Cellulose NF | 25 - 35 |
| Corn Starch NF | 10 - 12 |
| Talc USP | 2- 5 |

13. A pharmaceutical composition of any preceding claim which initially contains less than 1% by weight of N-formyldescarbonylethoxyloratadine.

## Patentansprüche

1. Pharmazeutische Zusammensetzung zur oralen Verabreichung umfassend eine anti-allergisch effektive Menge Descarbonylethoxyloratadin als der aktive Inhaltsstoff in einem pharmazeutisch akzeptablen Trägermedium umfassend eine DCL-schützende Menge eines pharmazeutisch akzeptablen Basissalzes und mindestens eines pharmazeutisch annehmbaren Zersetzungsmittels, worin das G/G-Verhältnis der DCL-Schutzmenge des pharmazeutisch annehmbaren basischen Salzes zu dem Zersetzungsmittel in dem Bereich von 1:1 bis 2:1, vorzugsweise 1,5:1 bis 2:1, und vorzugsweise 1,25:1 bis 1,75:1 liegt.

2. Pharmazeutische Zusammensetzung nach Anspruch 1, worin das mindestens eine pharmazeutisch akzeptable Zersetzungsmittel in einer Menge vorhanden ist, die zur Auflösung von mindestens 80 Gew.% der pharmazeutischen Zusammensetzung in 45 Minuten, in Übereinstimmung mit dem USP-Paddle-Auflösungstest <711> auf Seiten 1791-1793 von USP 23/NF 18, 1995, UNITED STATES PHARMACOPEIAL CONVENTION, INC., Rockvile MD 20852, ausreichend ist.

3. Pharmazeutische Zusammensetzung nach Anspruch 1, worin das pharmazeutisch akzeptable basische Salz ein Calcium-, Magnesium- oder Aluminiumsalz, oder deren Mischungen ist.

4. Pharmazeutische Zusammensetzung nach Anspruch 1, worin das pharmazeutisch akzeptable Basissalz ein Calciumphosphatsalz ist.

5. Pharmazeutische Zusammensetzung nach Anspruch 1, worin das pharmazeutisch akzeptable Trägermedium im wesentlichen frei von sauren Hilfsstoffen ist.

6. Pharmazeutische Zusammensetzung nach Anspruch 1, die weniger als 1 Gew.% N-Formyldescarbonylethoxyloratadin nach Lagerung bei 25°C und 60 % relativer Feuchtigkeit für mindestens 24 Monate enthält.

7. Pharmazeutische Zusammensetzung nach Anspruch 1, worin das G/G-Verhältnis der DCL-schützenden Menge des pharmazeutisch akzeptablen basischen Salzes zu der antiallergisch effektiven Menge Descarbonylethoxyloratadin der Bereich von 5:1 bis 60:1, vorzugsweise 7:1 bis 11:1, und insbesondere bevorzugt 10:1 bis 11:1 ist.

8. Pharmazeutische Zusammensetzung nach Ansprüchen 2 oder 6, umfassend eine DCL-schützende Menge von zweiwertem Calciumphosphat, eine Menge von mikrokristalliner Cellulose und von Stärke, die ausreicht, eine Auflösung von mindestens 80 Gew.% der pharmazeutischen Zusammensetzung in 45 Minuten, in Übereinstimmung mit dem USP-Paddle-Auflösungstest <711> auf Seiten 1791-1793 von USP 23/NF 18, 1995, UNITED STATES PHARMACOPEIAL CONVENTION, INC., Rockvile MD 20852, zu bewirken.

9. Pharmazeutische Zusammensetzung nach Anspruch 8, die umfaßt:
| Bestandteil | Menge (Gew.%) |
|---|---|
| Descarbonylethoxyloratadin | 0,5-15 |
| Dicalciumorthophosphat- | |
| Dihydrat USP | 10-90 |
| Mikrokristalline Cellulose NF | 5-60 |
| Maisstärke NF | 1-60 |
| Talkum USP | 0,5-20 |

10. Pharmazeutische Zusammensetzung nach Anspruch 8, die umfaßt:
| Bestandteil | Menge (Gew.%) |
|---|---|
| Descarbonylethoxyloratadin | 1-10 |
| Dicalciumorthophosphat- | |
| Dihydrat USP | 50-56 |
| Mikrokristalline Cellulose NF | 25-35 |
| Maisstärke NF | 10-12 |
| Talkum USP | 2-5 |

11. Pharmazeutische Zusammensetzung nach einem der vorhergehenden Ansprüche, worin die Menge an Descarbonylethoxyloratadin in dem Bereich von 1 bis 10 Gew.% liegt.

12. Pharmazeutische Zusammensetzung nach Anspruch 8, die umfaßt:
| Bestandteil | Menge (Gew.%) |
|---|---|
| Descarbonylethoxyloratadin | 1-10 |
| Dicalciumorthophosphat- | |
| Dihydrat USP | 50-56 |
| Mikrokristalline Cellulose NF | 25-35 |
| Maisstärke NF | 10-12 |
| Talkum USP | 2-5 |

13. Pharmazeutische Zusammensetzung nach einem der vorhergehenden Ansprüche, die anfangs weniger als 1 Gew.% N-Formyldescarbonylethoxyloratadin enthält.

## Revendications

1. Composition pharmaceutique conçue pour être administrée par voie orale, comprenant, en tant qu'ingrédient actif, de la décarbonyléthoxy-loratadine (DCL), en une quantité à effet anti-allergique, dans un milieu véhicule pharmacologiquement admissible qui comprend un sel basique pharmacologiquement admissible, en une quantité à effet de protection de la DCL, et au moins un agent désintégrant pharmacologiquement admissible, dans laquelle composition le rapport poids/poids de la quantité à effet de protection de la DCL du sel basique pharmacologiquement admissible audit désintégrant se situe dans l'intervalle allant de 1/1 à 2/1, et de préférence de 1,5/1 à 2/1 ou de préférence de 1,25/1 à 1,75/1.

2. Composition pharmaceutique conforme à la revendication 1, dans laquelle l'agent désintégrant pharmacologiquement admissible au nombre d'au moins un se trouve présent en une quantité suffisante pour provoquer la dissolution, en 45 minutes, d'au moins 80 % en poids de la composition pharmaceutique, d'après le test USP de dissolution sous agitation décrit sous <711> en pages 1791-1793 du document USP 23 / NF 18, 1995, United States Pharmacopeial Convention, Inc., Rockville MD 20852.

3. Composition pharmaceutique conforme à la revendication 1, dans laquelle le sel basique pharmacologiquement admissible est un sel de calcium, de magnésium ou d'aluminium, ou un mélange de tels sels.

4. Composition pharmaceutique conforme à la revendication 1, dans laquelle le sel basique pharmacologiquement admissible est un phosphate de calcium.

5. Composition pharmaceutique conforme à la revendication 1, dans laquelle le milieu véhicule pharmacologiquement admissible ne contient pratiquement pas d'excipients acides.

6. Composition pharmaceutique conforme à la revendication 1, qui contient moins d'environ 1 % en poids de N-formyl-décarbonyléthoxy-loratadine au bout d'au moins 24 mois de stockage à 60 % d'humidité relative et à 25 °C.

7. Composition pharmaceutique conforme à la revendication 1, dans laquelle le rapport poids/poids de la quantité à effet de protection de la DCL du sel basique pharmacologiquement admissible à la quantité à effet anti-allergique de la décarbonyléthoxy-loratadine vaut de 5/1 à 60/1, de préférence de 7/1 à 11/1, et mieux encore de 10/1 à 11/1.

8. Composition pharmaceutique conforme à l'une des revendications 2 à 6, qui comprend de l'hydrogénophosphate de calcium, en une quantité à effet de protection de la DCL, et de la cellulose microcristalline et de l'amidon, en quantité suffisante pour provoquer la dissolution, en 45 minutes, d'au moins 80 % en poids de la composition pharmaceutique, d'après le test USP de dissolution sous agitation décrit sous <711> en pages 1791-1793 du document USP 23 / NF 18, 1995, United States Pharmacopeial Convention, Inc., Rockville MD 20852.

9. Composition pharmaceutique conforme à la revendication 8, qui comprend :
| Ingrédient | Quantité (% en poids) |
|---|---|
| Décarbonyléthoxy-loratadine | 0,5 - 15 |
| Hydrogénophosphate de calcium dihydraté (USP) | 10 - 90 |
| Cellulose microcristalline NF | 5 - 60 |
| Amidon de maïs NF | 1 - 60 |
| Talc USP | 0,5 - 20 |

10. Composition pharmaceutique conforme à la revendication 8, qui comprend :
| Ingrédient | Quantité (% en poids) |
|---|---|
| Décarbonyléthoxy-loratadine | 0,5 - 15 |
| Hydrogénophosphate de calcium dihydraté (USP) | 45 - 60 |
| Cellulose microcristalline NF | 20 - 40 |
| Amidon de maïs NF | 5 - 15 |
| Talc USP | 1 - 10 |

11. Composition pharmaceutique conforme à l'une des revendications précédentes, dans laquelle la proportion pondérale de décarbonyléthoxy-loratadine vaut de 1 à 10 %.

12. Composition pharmaceutique conforme à la revendication 8, qui comprend :
| Ingrédient | Quantité (% en poids) |
|---|---|
| Décarbonyléthoxy-loratadine | 1 - 10 |
| Hydrogénophosphate de calcium dihydraté (USP) | 50 - 56 |
| Cellulose microcristalline NF | 25 - 35 |
| Amidon de maïs NF | 10 - 12 |
| Talc USP | 2 - 5 |

13. Composition pharmaceutique conforme à l'une des revendications précédentes, qui contient initialement moins de 1 % en poids de N-formyl-décarbonyléthoxy-loratadine.
